# EUROPEAN PATENT SPECIFICATION

(11) **EP 1 359 905 B1**
(45) Date of publication and mention of the grant of the patent: **26.07.2006**
(21) Application number: 02711840.5
(22) Date of filing: 06.02.2002
(51) Int. Cl.: A61K 31/00, A61K 31/44, A61K 31/495, A61K 31/444, A61P 17/04

(54) **USE OF MGLUR5 ANTAGONISTS FOR THE TREATMENT OF PRURITIC CONDITIONS**
VERWENDUNG VON MGLUR5 ANTAGONISTEN ZUR BEHANDLUNG VON PRURITISCHEN ZUSTÄNDEN
UTILISATION D'ANTAGONISTES MGLUR5 POUR LE TRAITEMENT D'AFFECTIONS PRURIGINEUSES

(30) Priority: 07.02.2001 GB 0103045
(43) Date of publication of application: 12.11.2003
(73) Proprietor: Novartis AG, 4056 Basel (CH); Novartis Pharma GmbH, 1230 Wien (AT)
(72) Inventor: GASPARINI, Fabrizio, CH-4415 Lausen (CH); URBAN, Laszlo, c/o The Novartis Inst., London, WC1E 6BN (GB); MEINGASSNER, Josef, Gottfried, A-2380 Perchtoldsdorf (AT)
(74) Representative: de Weerd, Petrus G.W.
(86) International application number: PCT/EP2002/001250
(87) International publication number: WO 2002/062323

(56) References cited:
- EP-A- 0 436 398
- WO-A-99/02497
- US-A- 5 498 795
- US-A- 5 534 641
- US-A- 5 574 036

## Description

The present invention relates to a new pharmaceutical use of compounds having antagonistic activity at metabotropic glutamate receptors (mGluRs).

Glutamate is the principal excitatory transmitter in the central nervous system acting through ionotropic glutamate receptors. It also plays a major role in activating modulatory pathways through the mGluRs.

Based on their amino acid sequence homology, agonist pharmacology and coupling to transduction mechanisms, the 8 presently known mGluR sub-types are classified into three groups. Group I receptors (mGluR1 and mGluR5) have been shown to be coupled to stimulation of phospholipase C resulting in phosphoinositide hydrolysis and elevation of intracellular Ca⁺⁺ levels, and, in some expression systems, to couple to modulation of ion channels, such as K⁺ channels, Ca⁺⁺ channels, non-selective cation channels or NMDA receptors. Group II receptors (mGluR2 and mGluR3) and Group III receptors (mGluRs 4, 6, 7 and 8) are negatively coupled to adenylylcyclase and have been shown to couple to inhibition of cAMP formation when heterologously expressed in mammalian cells, and to G-protein-activated inward rectifying potassium channels in Xenopus oocytes and in unipolar brush cells in the cerebellum.

Said mGluRs have been implicated as potentially important therapeutic targets for a number of neurological and psychiatric disorders largely based on studies with compounds not discriminating between mGluR subtypes (for review see Knopfel et al., J. Med. Chem. 38, 1417-26, 1995; Conn and Pin, Annu. Rev. Pharmacol. Toxicol. 37, 205-37, 1997). Particularly, for group I mGluR, the elucidation of the role of the individual receptor subtypes has been significantly hampered by the lack of potent, systemically active, subtype-selective compounds.

According to the present invention it has unexpectedly been found that mGluR5 antagonists, particularly selective mGluR5 antagonists, provide highly effective treatment of pruritic conditions.

These findings are based on experiments performed with compounds which display a high degree of selectivity and affinity as antagonists of the human and rat mGluR5 (selective mGluR5 antagonists). Selective mGluR5 antagonists, as used herein, typically exhibit about 100 fold greater activity at an mGluR5 receptor than at an mGluR1 receptor, preferably about 200 fold greater activity and most preferably about 400 fold greater activity.

Selective mGluR5 antagonists include 2-arylalkenyl-, 2-heteroarylalkenyl-, 2-arylalkynyl-, 2-heteroaryl-alkynyl-, 2-arylazo- and 2-heteroarylazo- pyridines, more particularly 6-methyl-2-(phenylazo)-3-pyridinol, (E)-2-methyl-6-styryl-pyridine and compounds of formula I 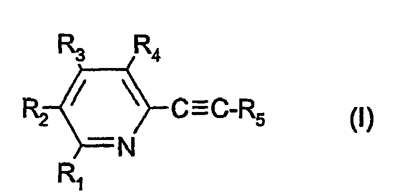 wherein
- R₁: is hydrogen, (C₁₋₄) alkyl, (C₁₋₄)alkoxy, cyano, ethynyl or di(C₁₋₄)alkylamino,
- R₂: is hydrogen, hydroxy, carboxy, (C₁₋₄) alkoxycarbonyl, di(C₁₋₄)alkylaminomethyl, 4-(4-fluoro-benzoyl)-piperidin-1-yl-carboxy, 4-t.-butyloxycarbonyl-piperazin-1-yl-carboxy, 4-(4-azido-2-hydroxybenzoyl)-piperazin-1-yl-carboxy or 4-(4-azido-2-hydroxy-3-iodo-benzoyl)-piperazin-1-yl-carboxy,
- R₃: is hydrogen, (C₁₋₄) alkyl, carboxy, (C₁₋₄)alkoxycarbonyl, (C₁₋₄)alkylcarbamoyl, hydroxy(C₁₋₄)alkyl, di(C₁₋₄)alkylaminomethyl, morpholinocarbonyl or 4-(4-fluoro-benzoyl)-piperidin-1-yl-carboxy,
- R₄: is hydrogen, hydroxy, carboxy, (C₂₋₅)alkanoyloxy, (C₁₋₄)alkoxycarbonyl, amino (C₁₋₄)alkoxy, di(C₁₋₄)alkylamino(C₁₋₄)alkoxy, di(C₁₋₄)alkylamino(C₁₋₄)alkyl or hydroxy(C₁₋₄)alkyl, and
- R₅: is a group of formula 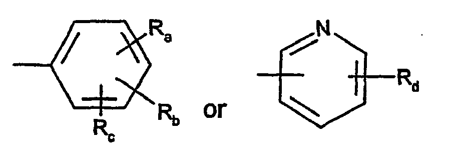 wherein
Rₐ and R_{b} independently are hydrogen, halogen, nitro, cyano, (C₁₋₄)alkyl, (C₁₋₄)alkoxy, trifluoromethyl, trifluoromethoxy or (C₂₋₅)alkynyl, and
R_{c} is hydrogen, fluorine, chlorine bromine, hydroxy (C₁₋₄)alkyl, (C₂₋₅)alkanoyloxy, (C₁₋₄)alkoxy or cyano, and
R_{d} is hydrogen, halogen or (C₁₋₄)alkyl,
in free form or in form of a pharmaceutically acceptable salt.

More particularly the findings are based on experiments performed with compounds including 2-[2-(pyridin-3-yl)ethynyl]-6-methyl-pyridine, 2-methyl-6-(phenylethynyl)-pyridine and 2-(3-fluoro-phenylethynyl)-6-methyl pyridine.

The compounds of formula I, their preparation and their use as selective mGluR5 antagonists are disclosed e.g. in WO 99/02 497.

Selective mGluR5 antagonists further include compounds of formula II 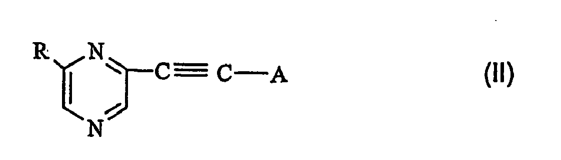 wherein
- R: is hydrogen or (C₁₋₄)alkyl and
- A: is a group of formula 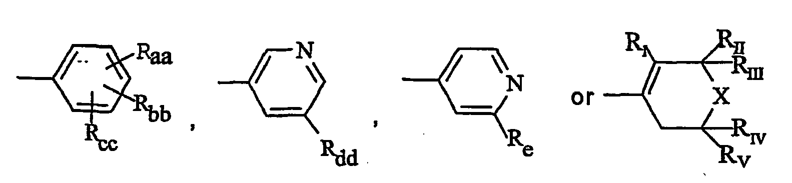 wherein
Rₐₐ, R_{bb} and R_{cc}, independently, are hydrogen, (C₁₋₄)alkyl, (C₁₋₄) alkoxy, hydroxy, hydroxy(C₁₋₄)alkyl, cyano or halo,
R_{dd} is cyano or halo,
Rₑ is hydroxy, (C₁₋₄)alkyl or (C₁₋₄)alkoxy,
R_{I} is hydrogen or (C₁₋₄)alkyl,
R_{II} and R_{III} each are hydrogen or form together a group oxo, =CH-CN, =N- OH, =N-O-(C₁₋₄)alkyl, =CH-PO₃[(C₁₋₄)alkyl]₂ or =CH-CO-R_{f} wherein R_{f} is (C₁₋₄)alkoxy or -NR_{g}Rₕ, Rg and Rₕ independently being hydrogen, (C₁₋₄)alkyl or phenyl,
R_{IV} and R_{V} independently are hydrogen, (C₁₋₄)alkyl or phenyl, and
X is (CH₂)ₙ, n being 0, 1 or 2,
CHR_{I}, R_{I} being hydroxy, (C₁₋₄)alkyl, (C₁₋₄)alkoxy, hydroxy(C₁₋₄)alkyl, (C₁₋₄)alkoxy(C₁₋₄)alkyl, (C₁₋₄)alkoxycarbonyl, carbamoyl, (C₁₋₄)alkylcarbamoyl, phenyl, pyridyl, thienyl or (Rⱼ, Rₖ)N-(C₁₋₄)alkyl, Rⱼ being hydrogen, or (C₁₋₄)alkyl, (C₁₋₄)alkanoyl or benzoyl and Rₖ being hydrogen or (C₁₋₄)alkyl, or, if R_{II} and R_{III} each are hydrogen, X can also be
NR_{I}, R_{I} being (C₁₋₄)alkoxy-carbonyl, benzyloxycarbonyl, benzoyl, thienyl, (C₁₋₄)alkanoyl, carbamoyl, mono- or
di(C₁₋₄)alkylcarbamoyl or phenylcarbamoyl, any phenyl ring in R_{I} being optionally mono- or. disubstituted by halo, cyano, (C₁₋₄)alkyl or (C₁₋₄)alkoxy,
in free form or in form of a pharmaceutically acceptable salt.

The compounds of formula II can be prepared by reacting a compound of formula III

Y₁-A III

with a compound of formula IV 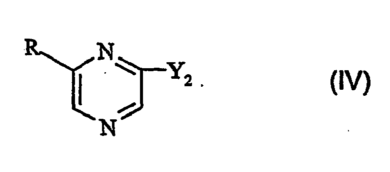 wherein R and A are as described above and one of Y₁ and Y₂ is a reactive esterified hydroxy group, e.g. trifluoro-methylsulfonyloxy, or halogen and the other is a group -C≡C-Y₃, Y₃ being hydrogen or a metallic group, whereby any functional group may be temporarily protected, and recovering the resulting group in free base or acid addition salt form.

When Y₃ is hydrogen, the condensation is preferably performed according to the Heck or Sonogashira coupling method. When Y₃ is a metallic group, tributylstannyl is suitably used. The starting materials of formulae III and IV are generally known.

Activity of mGluR5 antagonists as antipruritics according to the invention is evidenced for example in a model of magnesium deficiency-induced dermatosis in rat. This dermatosis is characterised by a transient erythematous maculopapular rash associated with a severe generalised pruritus. The animals scratch and bite themselves causing excoriations and wounds on the head and trunk (Neckermann G., Bavandi A., Meingassner J.G., Br. J. Dermatol; 2000; 142: 669-679). According to this model, male hairless rats (Ico:OFA hr/hr) obtained from Iffa Credo (Lyon, France) at an age of 3 weeks are maintained with a diet low in magnesium (C10350, Altromin, Lage, Germany) and demineralised water. After onset of symptoms, the test compound is applied orally to 5 rats per group on 5 consecutive days. Control animals are treated similarly with the vehicle alone. Efficacy is assessed by clinical examination and semiquantitative assessment of skin lesions. The intensity and extend of the erythematous maculopapular rash is scored with 0 (not present) to 4 (most severe changes, involvement of the whole trunk). The intensity of pruritus is evaluated by the scoring of excoriations at the head, shoulders, lateral abdomen/flanks and caudal dorsal trunk with 0 (not present), 1 (few lesions) or 3 (numerous lesions) resulting in a combined maximal score of 12 per animal. The animals are examined daily for 7 days after onset of treatment.

In this model, mGluR5 antagonists are found to inhibit signs of pruritus at daily doses of about 1 to about 100mg/kg/day. With 2-[2-(pyridin-3-yl)ethynyl]-6-methyl-pyridine, for example, oral treatment at 6 mg/fcg/day inhibits signs of pruritus whereas inflammatory skin reddening and infiltration is not inhibited.

Furthermore, the activity of mGluR5 antagonists as antipruritics is evidenced in a model of itch induced in mice by injection of the pruritic agent Compound 48/80 (Sigma, Catalog No. C 2313). The said pruritic agent when applied to the mouse skin induces scratching behaviour at the site of injection [Kuraishi et al., European Journal of Pharmacology 275: 229-233 (1995)].

Experiments are carried out on adult female and male C57BU6 mice (25-30 g). Individual mice receive the test compound p.o. (a vehicle alone for control animals) 30min. prior to subcutaneous injections of the above mentioned pruritic agent into the dorsal neck region and are then placed into a clear Perspex box. A maximum of 3 mice are closely and continuously observed by an experimenter for 30 minutes following injection. 'Scratching episodes' are defined as scratching focused at the site of injection using the hind paws, and differentiated from grooming behaviour that also involves licking and is systematically directed over all body regions. The duration of the itch behaviour is recorded by using a keyboard linked to three stop-clocks.

In this model, mGluR5 antagonists are found to decrease the number and duration of scratching episodes induced by the pruritic agent at doses of about 1 to 100mg/kg. 2-methyl-6-phenylethynyl-pyridine, for example, significantly decreases the duration of scratching episodes induced by 30µg/10µl s.c. of the pruritic agent following oral administration of 3-30mg/kg and 10-100mg/kg respectively.

These results indicate that mGluR5 antagonists are useful in the treatment of pruritic conditions.

In accordance with the above, the present invention provides:
a) the use of a mGluR5 antagonist for the treatment of pruritic conditions;
b) the use of a mGluR5 antagonist in the manufacture of a pharmaceutical composition for the treatment of pruritic conditions;
c) a pharmaceutical composition incorporating as active agent a mGluR5 antagonist for use in the treatment of pruritic conditions;
d) a method of treating pruritic conditions in a subject in need of such treatment, comprising administration to such subject of a therapeutically effective amount of a mGluR5 antagonist.

For the new uses according to the invention, the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 100 mg/kg body weight. In larger mammals, for example humans, an indicated daily dosage is in the range from about 5 to about 1000 mg of a compound for use according to the invention conveniently administered, for example, in divided doses up to five times a day.

The mGluR5 antagonist may be delivered orally for example in the form of tablets or capsules, or parenterally, e.g. by intravenous, intraperitoneal, intramuscular, subcutaneous, intranasal or intradermal injection, as well as by epicutaneous application (e.g. In a cream, ointment, gel or solution) or by transdermal application (e.g. with a lipid-soluble carrier in a skin patch placed on skin), or by gastrointestinal delivery (e.g., with a capsule or tablet). The preferred therapeutic compositions for inocula and dosage will vary with the clinical indication. The inocula is typically prepared from a dried mGluR5 antagonist preparation (e.g., a lyophilized powder) by suspending the preparation in a physiologically acceptable diluent such as water, saline, or phosphate-buffered saline.

Pharmaceutical compositions incorporating as active agent a mGluR5 antagonist are administered alone or in combination with pharmaceutically acceptable carriers, in either single or multiple doses. Suitable pharmaceutical carriers include inert solid diluents or fillers, sterile aqueous solutions, and various nontoxic organic solvents. The pharmaceutical compositions formed by combining the mGluR5 antagonist with the pharmaceutically acceptable carrier are then readily administered in a variety of dosage forms such as tablets, lozenges, syrups, injectable solutions, and the like. These pharmaceutical carriers can, if desired, contain additional ingredients such as flavorings, binders, excipients, and the like. Thus, for purposes of oral administration, tablets containing various excipients such as sodium citrate, calcium carbonate and calcium phosphate are employed along with various disintegrants such as starch, and preferably potato or tapioca starch, alginic acid, and certain complex silicates, together with binding agents such as polyvinylpyrolidone, sucrose, gelatin and acacia. Additionally, lubricating agents such as magnesium stearate, sodium lauryl sulfate and talc are often useful for tablefing purposes. Solid compositions of a similar type may also be employed as fillers in salt and hard filled gelatine capsules. Preferred materials for this purpose include lactose or milk sugar and high molecular weight polyethylene glycols. When aqueous suspensions of elixiers are desired for oral administration, the active mGluR5 antagonist is combined with various sweetening or flavoring agents, colored matter of dyes, and if desired, emulsifying or suspending agents, together with diluents such as water, ethanol, propylene glycol, glycerin and combinations thereof. For parenteral administration, solutions of the mGluR5 antagonist in sesame or peanut oil or in aqueous polypropylene glycol are employed, as well as sterile aqueous saline solutions of corresponding water soluble pharmaceutically acceptable metal salts. Such an aqueous solution should be suitably buffered if necessary and the liquid diluent first rendered isotonic with sufficient saline or glucose. These particular aqueous solutions are especially suitable for intravenous, intramuscular, subcutaneous and intraperitoneal injection. The sterile aqueous media employed are all readily obtainable by standard techniques well known to those skilled in the art. Additionally, it is possible to administer the aforesaid compounds topically (e.g. through a placed catheter) using an appropriate solution suitable for the purpose at hand.

Further embodiments of the invention provide articles of manufacture containing package inserts with instructions for therapeutic use, packaging material and a formulation of one or more of the mGluR5 antagonist containing pharmaceutical compositions. The instructions for use will commonly identify administering the mGluR5 antagonist to ameliorate one or more symptoms of a dysfunction having a pain and/or anxiety component. The article of manufacture Will also commonly contain a label indicating the compound, or composition, and its use for ameliorating one or more symptoms associated with the subject dysfunction.

The method of treating pruritic conditions in accordance with the invention is intended to mean a method of delivering to a subject in need thereof a pharmaceutical preparation of mGluR5 antagonist with the aim of treating or preventing one or more symptoms of a dysfunction having a pruritic condition component. The subject method includes delivering the preparation to a patient i) before the dysfunction has been diagnosed, e.g., prophylactic protocols delivered with the aim of preventing development of the dysfunction, as well as, ii) after the dysfunction has been diagnosed, e.g., therapeutic protocols.

In accordance with said method for treating pruritic conditions the mGluR5 antagonist is introduced in the structure of any medicinal form or composition. It is used as a solitary agent of medication or in combination with other medicinal preparations. Since the pharmacokinetics and pharmacodynamics of the mGluR5 antagonist will vary in different patients, the most preferred method for achieving a therapeutic concentration in a tissue is to gradually escalate the dosage and monitor the clinical effects. The initial dose, for such an escalating dosage regimen of therapy, will depend upon the route of administration.

Transdermal and epicutaneous administrations are preferred routes of administration. For transdermal administration, the mGluR5 antagonist may be administered in any conventional liquid or solid transdermal pharmaceutical composition, e.g. as described in Remington's Pharmaceutical Sciences 16th Edition Mack; Sucker, Fuchs and Spieser, Pharmazeutische Technologie 1st Edition, Springer and in GB 2098865 A or DOS 3212053.

## Claims

1. The use of a mGluR5 antagonist in the manufacture of a pharmaceutical composition for the treatment of prutritic conditions.

2. The use according to claim 1, wherein the mGluR5 antagonist is a compound of formula I 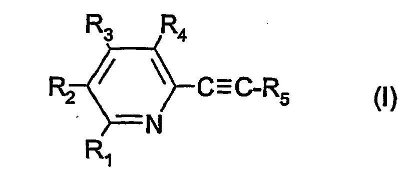 wherein
R₁ is hydrogen, (C₁₋₄) alkyl, (C₁₋₄)alkoxy, cyano, ethynyl or di(C₁₋₄)alkylamino,
R₂ is hydrogen, hydroxy, carboxy, (C₁₋₄) alkoxycarbonyl, di(C₁₋₄)alkylaminomethyl, 4-(4-fluoro-benzoyl)-piperidin-1-yl-carboxy, 4-t.-butyloxycarbonyl-piperazin-1-yl-carboxy, 4-(4-azido-2-hydroxybenzoyl)-piperazin-1-yl-carboxy or 4-(4-azido-2-hydroxy-3-iodo-benzoyl)-piperazin-1-yl-carboxy,
R₃ is hydrogen, (C₁₋₄) alkyl, carboxy, (C₁₋₄)alkoxycarbonyl, (C₁₋₄)alkylcarbamoyl, hydroxy(C₁₋₄)alkyl, di(C₁₋₄)alkylaminomethyl, morpholinocarbonyl or 4-(4-fluorobenzoyl)-piperidin-1-yl-carboxy,
R₄ is hydrogen, hydroxy, carboxy, (C₂₋₅)alkanoyloxy, (C₁₋₄)alkoxycarbonyl, amino (C₁₋₄)alkoxy, di(C₁₋₄)alkylamino(C₁₋₄)alkoxy, di(C₁₋₄)alkylamino(C₁₋₄)alkyl or hydroxy(C₁₋₄)alkyl, and
R₅ is a group of formula 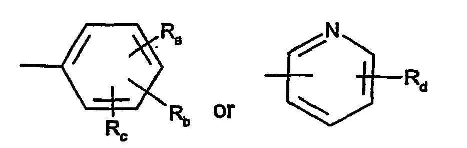 wherein
Rₐ and R_{b} independently are hydrogen, halogen, nitro, cyano, (C₁₋₄)alkyl, (C₁₋₄)alkoxy, trifluoromethyl, trifluoromethoxy or (C₂₋₅)alkynyl, and
R_{c} is hydrogen, fluorine, chlorine bromine, hydroxy (C₁₋₄)alkyl, (C₂₋₅)alkanoyloxy, (C₁₋₄)alkoxy or cyano, and
R_{d} is hydrogen, halogen or (C₁₋₄)alkyl,
in free form or in form of a pharmaceutically acceptable salt.

3. The use according to claim 1, wherein the mGluR5 antagonist is a compound of formula II 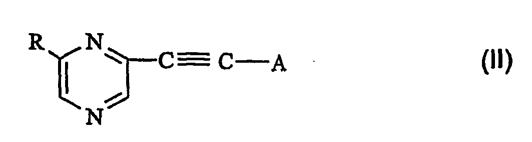 wherein
R is hydrogen or (C₁₋₄)alkyl and
A is a group of formula 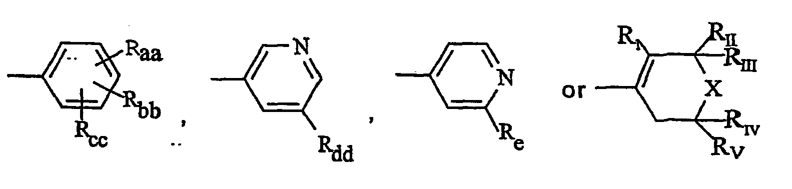 wherein
Rₐₐ, R_{bb} and R_{cc}, independently, are hydrogen, (C₁₋₄)alkyl, (C₁₋₄) alkoxy, hydroxy, hydroxy(C₁₋₄)alkyl, cyano or halo,
R_{dd} is cyano or halo,
Rₑ is hydroxy, (C₁₋₄)alkyl or (C₁₋₄)alkoxy,
R_{I} is hydrogen or (C₁₋₄)alkyl,
R_{II} and R_{III} each are hydrogen or form together a group oxo, =CH-CN, =N- OH, =N-O-(C₁₋₄)alkyl, =CH-PO₃[(C₁₋₄)alkyl]₂ or =CH-CO-R_{f} wherein R_{f} is (C₁₋₄)alkoxy or -NR_{g}Rₕ, R_{g} and Rₕ independently being hydrogen, (C₁₋₄)alkyl or phenyl,
R_{IV} and R_{V} independently are hydrogen, (C₁₋₄)alkyl or phenyl, and
X is (CH₂)ₙ, n being 0, 1 or 2,
CHRᵢ, Rᵢ being hydroxy, (C₁₋₄)alkyl, (C₁₋₄)alkoxy, hydroxy(C₁₋₄)alkyl, (C₁₋₄)alkoxy(C₁₋₄)alkyl, (C₁₋₄)alkoxycarbonyl, carbamoyl, (C₁₋₄)alkylcarbamoyl, phenyl, pyridyl, thienyl or (Rⱼ, Rₖ)N-(C₁₋₄)alkyl, Rⱼ being hydrogen, or (C₁₋₄)alkyl, (C₁₋₄)alkanoyl or benzoyl and Rₖ being hydrogen or (C₁₋₄)alkyl, or, if R_{II} and R_{III} each are hydrogen, X can also be
NR_{I}, R_{I} being (C₁₋₄)alkoxy-carbonyl, benzyloxycarbonyl, benzoyl, thienyl, (C₁₋₄)alkanoyl, carbamoyl, mono- or di(C₁₋₄)alkylcarbamoyl or phenylcarbamoyl, any phenyl ring in R_{I} being optionally mono- or. disubstituted by halo, cyano, (C₁₋₄)alkyl or (C₁₋₄)alkoxy,
in free form or in form of a pharmaceutically acceptable salt.

4. The use according to claim 1, wherein the mGluR5 antagonist is 2-[2-(pyridine-3-yl)ethynyl]-6-methyl-pyridine, in free form or in form of a pharmaceutically acceptable salt.

## Patentansprüche

1. Verwendung eines mGluR5-Antagonisten bei der Herstellung einer pharmazeutischen Zusammensetzung für die Behandlung von pruritischen Zuständen.

2. Verwendung nach Anspruch 1, wobei der mGluR5-Antagonist für eine Verbindung der Formel I 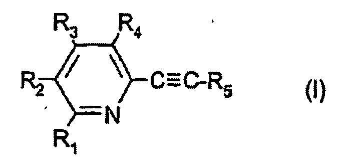 steht, wobei
R₁ für Wasserstoff, (C₁-C₄)-Alkyl, (C₁-C₄)-Alkoxy, Cyano, Ethinyl oder Di-(C₁-C₄)-alkylamino steht,
R₂ für Wasserstoff, Hydroxy, Carboxy, (C₁-C₄)-Alkolxycarbonyl, Di-(C₁-C₄)-alkylaminomethyl, 4-(4-Fluorbenzoyl)-piperidin-1-ylcarboxy, 4-tert.-Butyloxycarbonylpiperazin-1-ylcarboxy, 4-(4-Azido-2-hydroxybenzoyl)-piperazin-1-ylcarboxy oder 4-(4-Azido-2-hydroxy-3-iodbenzoyl)-piperazin-1-ylcarboxy steht,
R₃ für Wasserstoff, (C₁-C₄)-Alkyl, Carboxy, (C₁-C₄)-Alkoxycarbonyl, (C₁-C₄)-Alkylcarbamoyl, Hydroxy-(C₁-C₄)-alkyl, Di-(C₁-C₄)-alkylaminomethyl, Morpholinocarbonyl oder 4-(4-Fluorbenzoyl)-piperidin-1-ylcarboxy steht,
R₄ für Wasserstoff, Hydroxy, Carboxy, (C₂-C₅)-Alkanoyloxy, (C₁-C₄)-Alkoxycarbonyl, Amino-(C₁-C₄)-alkoxy, Di-(C₁-C₄)-alkylamino-(C₁-C₄)-alkoxy, Di-(C₁-C₄)-alkylamino-(C₁-C₄)-alkyl oder Hydroxy-(C₁₋C₄)-alkyl steht, und
R₅ für eine Gruppe der Formel 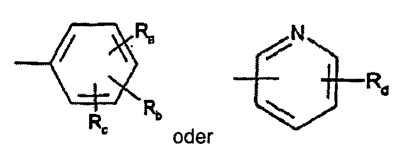 steht, wobei
Rₐ und R_{b} unabhängig für Wasserstoff, Halogen, Nitro, Cyano, (C₁-C₄)-Alkyl, (C₁-C₄)-Alkoxy, Trifluormethyl, Trifluormethoxy oder (C₂-C₅)-Alkinyl stehen, und
R_{c} für Wasserstoff, Fluor, Chlor, Brom, Hydroxy-(C₁-C₄)-alkyl, (C₂-C₅)-Alkanoyloxy, (C₁-C₄)-Alkoxy oder Cyano steht, und
R_{d} für Wasserstoff, Halogen oder (C₁-C₄)-Alkyl steht,
in freier Form oder in Form eines pharmazeutisch annehmbaren Salzes.

3. Verwendung nach Anspruch 1, wobei der mGluR5 Antagonist für eine Verbindung der Formel II 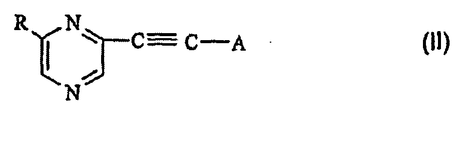 steht, wobei
R für Wasserstoff oder (C₁-C₄)-Alkyl steht, und
A für eine Gruppe der Formel 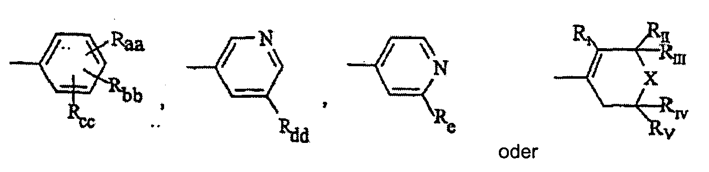 steht, wobei
Rₐₐ, R_{bb} und R_{cc} unabhängig für Wasserstoff, (C₁-C₄)-Alkyl, (C₁-C₄)-Alkoxy, Hydroxy, Hydroxy-(C₁₋C₄)-alkyl, Cyano oder Halogen stehen,
R_{dd} für Cyano oder Halogen steht,
Rₑ für Hydroxy, (C₁-C₄)-Alkyl oder (C₁-C₄)-Alkoxy steht,
Rₗ für Wasserstoff oder (C₁-C₄)-Alkyl steht,
R_{II} und R_{III} jeweils für Wasserstoff stehen oder zusammen für eine Oxo-Gruppe, =CH-CN, =N-OH, =N-O-(C₁-C₄)-Alkyl, =CH-PO₃[(C₁-C₄)-Alkyl]₂ oder =CH-CO-R_{f} stehen, wobei R_{f} für (C₁-C₄)-Alkoxy oder -NR₉Rₕ steht, Rg und Rₕ unabhängig für Wasserstoff, (C₁-C₄)-Alkyl oder Phenyl stehen,
R_{IV} und R_{V} unabhängig für Wasserstoff, (C₁-C₄)-Alkyl oder Phenyl stehen, und
X steht für (CH₂)ₙ, wobei n für 0, 1 oder 2 steht,
CHRᵢ, wobei Rᵢ für Hydroxy, (C₁-C₄)-Alkyl, (C₁-C₄)-Alkoxy, Hydroxy-(C₁-C₄)-alkyl, (C₁-C₄)-Alkoxy-(C₁-C₄)-alkyl, (C₁-C₄)-Alkoxycarbonyl, Carbamoyl, (C₁-C₄)-Alkylcarbamoyl, Phenyl, Pyridyl, Thienyl oder (Rⱼ, Rₖ)N-(C₁-C₄)-Alkyl steht, wobei Rⱼ für Wasserstoff oder (C₁-C₄)-Alkyl, (C₁-C₄)-Alkanoyl oder Benzoyl steht und Rₖ für Wasserstoff oder (C₁-C₄)-Alkyl steht, oder, wenn R_{II} und R_{III} jeweils für Wasserstoff stehen, kann X auch
NR_{I} sein, wobei R_{I} für (C₁-C₄)-Alkoxycarbonyl, Benzoyloxycarbonyl, Benzoyl, Thienyl, (C₁-C₄)-Alkanoyl, Carbamoyl, Mono- oder Di-(C₁-C₄)-alkyl-carbamoyl oder Phenylcarbamoyl steht, jeder beliebige Phenylring in R_{I} gegebenenfalls mono- oder disubstituiert sein kann durch Halogen, Cyano, (C₁-C₄)-Alkyl oder (C₁-C₄)-Alkoxy,
in freier Form oder in Form eines pharmazeutisch annehmbaren Salzes.

4. Verwendung nach Anspruch 1, wobei der mGluR5-Antagonist für 2-[2-(Pyridin-3-yl)ethinyl]-6-methylpyridin in freier Form oder in Form eines pharmazeutisch annehmbaren Salzes steht.

## Revendications

1. Utilisation d'un antagoniste de mGluR5 dans la fabrication d'une composition pharmaceutique destinée au traitement d'affections prurigineuses.

2. Utilisation selon la revendication 1, dans laquelle l'antagoniste de mGluR5 est un composé de formule 1 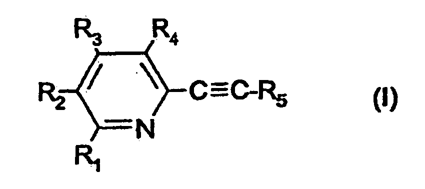 dans laquelle
R₁ est un atome d'hydrogène, un groupe alkyle en C₁₋₄, alcoxy en C₁₋₄, cyano, éthynyle ou di(alkyl en C₁₋₄)amino,
R₂ est un atome d'hydrogène, un groupe hydroxy, carboxy, alcoxy(en C₁₋₄)caronyle, di(alkyl en C₁₋₄)aminométhyle, 4-(4-fluorobenzoyl)pipéridin-1-yl-carboxy, 4-t-butyloxycarbonylpipérazin-1-yl-carboxy, 4-(4-azido-2-hydroxybenzoyl)pipérazin-1-yl-carboxy ou 4-(4-azido-2-hydroxy-3-iodobenzoyl)pipérazin-1-yl-carboxy,
R₃ est un atome d'hydrogène, un groupe alkyle en C₁₋₄, carboxy, alcoxy(en C₁₋₄)carbonyle, alkyl(en C₁₋₄)carbamoyle, hydroxy(alkyle en C₁₋₄), di(alkyl en C₁₋₄)aminométhyle, morpholinocarbonyle ou 4-(4-fluorobenzoyl)pipéridin-1-yl-carboxy,
R₄ est un atome d'hydrogène, un groupe hydroxy, carboxy, alcanoyloxy en C₂₋₅, alcoxy(en C₁₋₄)carbonyle, amino(alcoxy en C₁₋₄), di(alkyl en C₁₋₄)amino(alcoxy en C₁₋₄), di(alkyl en C₁₋₄)amino(alkyle en C₁₋₄) ou hydroxy(alkyle en C₁₋₄), et
R₅ est un groupe de formule 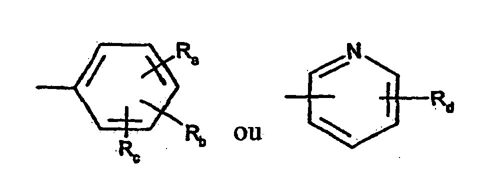 où
Rₐ et R_{b} représentent indépendamment un atome d'hydrogène, d'halogène, un groupe nitro, cyano, alkyle en C₁₋₄, alcoxy en C₁₋₄, trifluorométhyle, trifluorométhoxy ou alcynyle en C₂₋₅, et
R_{c} est un atome d'hydrogène, de fluor, de chlore, de brome, un groupe hydroxy(alkyle en C₁₋₄), alcanoyloxy en C₂₋₅, alcoxy en C₁₋₄ ou cyano, et
R_{d} est un atome d'hydrogène, d'halogène ou un groupe alkyle en C₁₋₄,
sous forme libre ou sous forme d'un sel acceptable sur le plan pharmaceutique.

3. Utilisation selon la revendication 1, dans laquelle l'antagoniste de mGluR5 est un composé de formule II 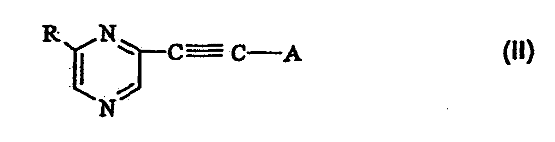 dans laquelle
R est un atome d'hydrogène ou un groupe alkyle en C₁₋₄ et
A est un groupe de formule 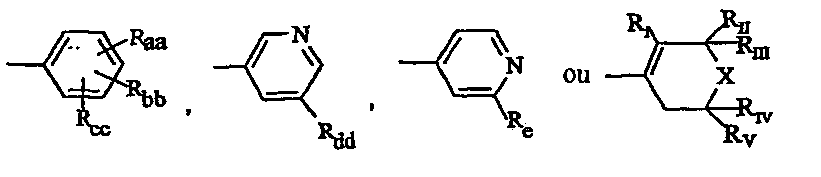 où
Rₐₐ, R_{bb} et R_{cc} représentent indépendamment un atome d'hydrogène, un groupe alkyle en C₁₋₄, alcoxy en C₁₋₄, hydroxy, hydroxy(alkyle en C₁₋₄), cyano ou halogéno,
R_{dd} est un cyano ou un halogéno,
Rₑ est un groupe hydroxy, alkyle en C₁₋₄ ou alcoxy en C₁₋₄,
R_{I} est un atome d'hydrogène ou un groupe alkyle en C₁₋₄,
R_{II} et R_{III} représentent chacun un atome d'hydrogène ou forment ensemble un groupe oxo, =CH-CN, =N-OH, =N-O-(alkyle en C₁₋₄), =CH-PO₃[alkyl(en C₁₋₄)]₂ ou =CH-CO-R_{f}
où R_{f} est un groupe alcoxy en C₁₋₄ ou -NR_{g}Rₕ, Rg et Rₕ étant indépendamment un atome d'hydrogène, un groupe alkyle en C₁₋₄ ou phényle,
R_{IV} et R_{V} représentent indépendamment un atome d'hydrogène, un groupe alkyle en C₁₋₄ ou phényle, et
X représente (CH₂)ₙ, n étant égal à 0,1 ou 2,
CHRᵢ, Rᵢ étant un groupe hydroxy, alkyle en C₁₋₄, alcoxy en C₁₋₄, hydroxy(alkyle en C₁₋₄), alcoxy(en C₁₋₄)alkyle en C₁₋₄, alcoxy(en C₁₋₄)carbonyle, carbamoyle, alkyl(en C₁₋₄)carbamoyle, phényle, pyridyle, thiényle ou (Rⱼ, Rₖ)N-(alkyle en C₁₋₄), Rⱼ étant un atome d'hydrogène, ou un groupe alkyle en C₁₋₄, alcanoyle en C₁₋₄ ou benzoyle et Rₖ étant un atome d'hydrogène ou un groupe alkyle en C₁₋₄, ou bien, si R_{II} et R_{III} représentent chacun un atome d'hydrogène, X peut être également
NR₁ R₁ étant un groupe alcoxy(en C₁₋₄)carbonyle, benzyloxycarbonyle, benzoyle, thiényle, alcanoyle en C₁₋₄, carbamoyle, mono- ou di(alkyl en C₁₋₄)carbamoyle ou phénylcarbamoyle, tout cycle phényle dans R₁ étant éventuellement mono- ou disubstitué par un halogèno, un cyano, un groupe alkyle en C₁₋₄ ou alcoxy en C₁₋₄,
sous forme libre ou sous forme d'un sel acceptable sur le plan pharmaceutique.

4. Utilisation selon la revendication 1, dans laquelle l'antagoniste de mGluR5 est la 2-[2-(pyridine-3-yl)éthynyl]-6-méthyl-pyridine, sous forme libre ou sous forme d'un sel acceptable sur le plan pharmaceutique.
